# EUROPÄISCHE PATENTSCHRIFT

(11) **EP 0 839 167 B1**
(45) Veröffentlichungstag und Bekanntmachung des Hinweises auf die Patenterteilung: **20.06.2001**
(21) Anmeldenummer: 97921795.7
(22) Anmeldetag: 26.04.1997
(51) Int. Cl.: C09C 1/00, C01G 23/00, C09D 7/12, C09D 11/00, A61K 7/00, C08K 3/00, C04B 33/14, C03C 4/02

(54) **TITANATHALTIGE PERLGLANZPIGMENTE**
TITANIUM-CONTAINING NACREOUS PIGMENTS
PIGMENTS A LUSTRE NACRE RENFERMANT DU TITANE

(30) Priorität: 09.05.1996 DE 19618563
(43) Veröffentlichungstag der Anmeldung: 06.05.1998
(73) Patentinhaber: MERCK PATENT GmbH, 64293 Darmstadt (DE)
(72) Erfinder: ANDES, Stephanie, D-63477 Maintal 3 (DE); HOCK, Sabine, D-64850 Schaafheim (DE); BRENNER, Günter, D-64347 Griesheim (DE); BRÜCKNER, Dieter, D-64289 Darmstadt (DE); HEYLAND, Andrea, D-64385 Ober-Kainsbach (DE); KUNTZ, Matthias, D-64342 Seeheim (DE); OSTERRIED, Karl, D-64807 Dieburg (DE); PFAFF, Gerhard, D-64839 Münster (DE); SCHMELZ, Michael, D-65830 Kriftel (DE)
(86) Internationale Anmeldenummer: EP9702168
(87) Internationale Veröffentlichungsnummer: WO9743348

(56) Entgegenhaltungen:
- EP-A- 0 045 851
- EP-A- 0 211 351
- EP-A- 0 307 747
- FR-A- 1 273 230
- DATABASE WPI Week 8706 Derwent Publications Ltd., London, GB; AN 87-039671 XP002037924 & JP 61 295 234 A (SUMITUMO CHEM. IND.) , 26.Dezember 1986

## Beschreibung

Die Erfindung betrifft sehr dünne Perlglanzpigmente auf der Basis von eisenoxidbeschichtetem plättchenförmigem Titandioxid.

Eisenoxidhaltige Perlglanzpigmente sind mehrfach beschrieben worden und werden seit vielen Jahren mit Erfolg angewendet. Dabei werden sowohl Pigmente beschrieben, bei denen Eisenoxid zusammen mit Titandioxid auf das Substrat aufgefällt wird, als auch Pigmente, bei denen die Fällungen hintereinander erfolgen. Beim nachfolgenden Glühen diffundiert Eisenoxid in die TiO₂-Schicht und es kommt zur Ausbildung von Pseudobrookit (Fe₂TiO₅). Als Substrat wird ausschließlich Glimmer verwendet.

In US 30 87 828 wird beschrieben, daß durch Abscheiden einer Fe₂O₃-Schicht auf eine TiO₂-Schicht goldfarbene Glimmerpigmente erhalten werden, die beim Glühen einen rötlichen Farbton annehmen.

In US 3 874 890 wird ein Verfahren zur Herstellung von goldfarbenen Perlglanzpigmenten beschrieben, bei dem ein mit TiO₂ und/oder ZrO₂ beschichtetes Glimmerpigment zunächst mit Eisen(II)-hydroxid beschichtet wird, das dann zu Fe₂O₃ oxidiert wird.

US 4 744 832 beschreibt ein Perlglanzpigment auf Basis von mit Metalloxiden überzogenen plättchenförmigen Substraten, insbesondere Glimmer, wobei die Metalloxidschicht sowohl Titan als auch Eisen enthält und das Pigment einen Mehrschichtaufbau besitzt, wobei auf eine erste Schicht von TiO₂ in der Rutilform eine Schicht von Pseudobrookit und eine Eisenoxidschicht folgt.

Glimmerpigmente werden in großem Umfang in der Druck- und Lackindustrie, in der Kosmetik und in der Kunststoffverarbeitung verwendet. Sie zeichnen sich durch Interferenzfarben und einen hohen Glanz aus. Für die Ausbildung extrem dünner Schichten sind aber Glimmerpigmente nicht geeignet, weil Glimmer als Substrat für die Metalloxidschichten des Pigmentes bereits eine Dicke von 200 bis 1200 nm aufweist. Nachteilig ist weiter hin, daß die Dicke der Glimmerplättchen innerhalb einer bestimmten Fraktion, die durch die Plättchengröße bestimmt wird, teilweise deutlich um einen Mittelwert schwankt. Außerdem handelt es sich bei Glimmer um ein natürlich vorkommendes Mineral, das durch Fremdionen verunreinigt ist. Desweiteren sind technisch sehr aufwendige und zeitraubende Aufbereitungsschritte notwendig. Hierzu zählen vor allem Mahlen und Klassieren.

Auf dicken Glimmerplättchen basierende, mit Metalloxiden umhüllte Perlglanzpigmente haben aufgrund der Dicke des Randes einen deutlichen Streuanteil, speziell bei feineren Korngrößenverteilungen unter 20 µm.

Als Ersatz für Glimmer sind dünne Glasplättchen vorgeschlagen worden, die durch Walzen einer Glasschmelze mit nachfolgendem Mahlen erhalten werden. Interferenzpigmente auf der Basis derartiger Materialien weisen zwar Farbeffekte auf, die denen herkömmlicher, auf Glimmer basierender Pigmente überlegen sind. Nachteilig ist jedoch, daß die Glasplättchen eine sehr große mittlere Dicke von etwa 10-15 µm und eine sehr breite Dickenverteilung (typischerweise zwischen 4 und 20 um) aufweisen, während die Dicke von Interferenzpigmenten typischerweise nicht größer als 3 µm ist.

In EP 0,384,596 wird ein Verfahren beschrieben, bei dem hydratisiertes Alkalisilikat bei Temperaturen von 480-500 °C mit einem Luftstrahl beaufschlagt wird, wobei sich Blasen mit dünnen Wandstärken bilden; die Blasen werden anschließend zerkleinert und man erhält plättchenförmige Alkalisilikatsubstrate mit einer Dicke von weniger als 3 µm. Das Verfahren ist jedoch aufwendig und die Dickenverteilung der erhaltenen Plättchen ist relativ breit.

In DE 11 36 042 ist ein kontinuierliches Bandverfahren zur Herstellung plättchen- oder flitterartiger Oxyde oder Oxydhydrate von Metallen der IV. und V. Gruppe sowie der Eisen-Gruppe des Periodensystems beschrieben. Dabei wird auf ein kontinuierliches Band ggf. zunächst eine Trennschicht aus z.B. Silikonlack aufgebracht, um das spätere Ablösen der Metalloxidschicht zu erleichtern. Anschließend wird ein Flüssigkeitsfilm aus einer Lösung einer hydrolysierbaren Verbindung des in das gewünschte Oxid umzuwandelnden Metalls aufgebracht, der Film wird getrocknet und anschließend mit einer Rüttelvorrichtung abgelöst. Die Schichtdicke der erhaltenen Plättchen wird mit 0,2 bis 2 pm angegeben, ohne hierfür konkrete Beispiele zu nennen.

In EP 0,240,952 und EP 0,236,952 ist ein kontinuierliches Bandverfahren zur Herstellung verschiedener plättchenförmiger Materialien, darunter auch Siliciumdioxid, Aluminiumoxid und Titandioxid vorgeschlagen worden. Dabei wird über ein Rollensystem auf ein glattes Band ein dünner flüssiger Film definierter Dickes eines Precursors des plättchenförmigen Materials aufgebracht; der Film wird getrocknet und von dem Band abgelöst, wobei sich plättchenförmige Teilchen bilden. Die Teilchen werden anschließend gegebenenfalls geglüht, gemahlen und klassiert.

Die Dicke der nach dem in EP 0 240 952 beschriebenen Verfahren erhaltenen Plättchen ist relativ gut definiert, da der Film z.B. auf das kontinuierliche Band über ein Rollensystem sehr gleichmäßig aufgebracht wird. Die Schichtdicke der Plättchen wird in den Beispielen mit 0,3 bis 3,0 µm angegeben. Gemäß Beispiel 1 wird eine erste Rolle mit dem verwendeten Precursor benetzt, indem man diese Rolle teilweise in einen mit dem Precursor befüllten Vorratsbehälter eintaucht. Der Film wird von dieser Rolle auf eine zweite, gleichsinnig rotierende Rolle übertragen, die mit der ersten in sehr engem Kontakt steht. Schließlich wird der Film von der zweiten Rolle auf das kontinuierliche Band abgerollt.

Nachteilig sind jedoch die Verwendung sehr teurer Precursormaterialien sowie insbesondere die erhöhten Anforderungen an die Arbeitsplatzsicherheit, die beim Einsatz metallorganischer Verbindungen gestellt werden müssen. Die vollständige chemische Umwandlung des Precursors in das gewünschte Schichtmaterial macht in der Regel eine starke Erhitzung des Filmes und des Bandmaterials erforderlich. Neben der dabei auftretenden erheblichen thermischen Belastung des Bandmaterials, wirken sich auch der hohe Energieaufwand und die Einschränkung der Prozeßgeschwindigkeit sehr nachteilig auf die Wirtschaftlichkeit des Verfahrens aus.

WO 93/08 237 beschreibt plättchenförmige Pigmente, bestehend aus einer plättchenförmigen Matrix aus Siliciumdioxid, die lösliche oder nichtlösliche Farbmittel enthalten kann und die mit einer oder mehreren reflektierenden Schichten aus Metalloxiden oder Metallen belegt ist. Die plättchenförmige Matrix wird durch Verfestigung von Wasserglas auf einem endlosen Band hergestellt.

In DE 1 273 098 wird die Herstellung eines Perlmuttpigmentes durch Aufdampfen von ZnS-, MgF₂-, ZnO-, CaF₂- und TiO₂-Filmen auf ein endloses Band beschrieben. Dieses Verfahren ist aber ebenso, wie
das in US 4 879 140 beschriebene Verfahren, bei dem plättchenförmige Pigmente mit Si- und SiO₂-Schichten durch Plasmaabscheidung aus SiH₄ und SiCl₄ erhalten werden, mit einem sehr hohen apparativen Aufwand verbunden.

Trotz zahlreicher Versuche konnte bisher noch kein wirtschaftliches Verfahren zur Herstellung sehr dünner plättchenförmiger Titandioxidpigmente mit einer Schichtdicke von kleiner als 500 nm entwickelt werden.

Aufgabe der Erfindung ist es, ein hochglänzendes titanhaltiges Perlglanzpigment mit einer Schichtdicke von kleiner als 500 nm und einer Schichtdickentoleranz von weniger als 10 % bereitzustellen.

Diese Aufgabe wird gemäß der Erfindung gelöst durch ein einschichtiges oder mehrschichtiges Perglanzpigment, bestehend aus Eisentitanat und gegebenenfalls Titanoxid und/oder Eisenoxid, erhältlich durch Verfestigung einer wäßrigen Lösung einer thermisch hydrolysierbaren Titanverbindung auf einem endlosen Band, Ablösung der entstandenen Schicht, Beschichtung der erhaltenen Titandixodiplättchen ohne Zwischentrockung im Naßverfahren mit Eisenoxid, Trocknung und Kalzination des erhaltenen Materials in einer oxidierenden oder reduzierenden Gasatmosphäre bei mindestens 700 °C.

Das Eisentitanat besteht entweder aus Pseudobrookit (Fe₂TiO₅) oder Ilmenit (FeTiO₃). Die wäßrige Lösung einer thermisch hydrolysierbaren Titanverbindung für die Herstellung der Titandioxidplättchen auf dem endlosen Band ist bevorzugt eine wäßrige Titantetrachloridlösung. Die Konzentration des Titansalzes in dieser Lösung beträgt 7 bis 30 Gew.-%, vorzugsweise 8 bis 15 Gew.-%.

Die erfindungsgemäßen Pigmente basieren auf plättchenförmigen Titandioxidpartikeln. Diese Plättchen haben eine Dicke zwischen 10 nm und 500 nm, vorzugsweise zwischen 40 und 150 nm. Die Ausdehnung in die beiden anderen Dimensionen beträgt zwischen 2 und 200 µm und insbesondere zwischen 5 und 50 µm.

Die Zusammensetzung und der Schichtaufbau des erfindungsgemäßen Pigmentes ist abhängig von der Dicke der als Substrat dienenden Titandixoidplättchen, der Schichtdicke des aufgebrachten Eisenoxids und Titandioxids und von den Kalzinierungsbedingungen.

Die Eisenoxidschicht und die Titandioxidschicht werden vorzugsweise naßchemisch nach bekannten Verfahren auf die Titandixoidplättchen aufgebracht. Weiterhin kann die Beschichtung der getrockneten Titandioxidplättchen auch in einem Wirbelbettreaktor durch Gasphasenbeschichtung erfolgen, wobei die in EP 0 045 851 und EP 0 106 235 zur Herstellung von Perlglanzpigmenten vorgeschlagenen Verfahren entsprechend angewendet werden können.

Bei einer ersten Ausführungsform besteht das Pigment nur aus Eisentitanat.

Bei einer zweiten Ausführungsform ist das Pigment zweischichtig, wobei der Kern aus plättchenförmigem Titandioxid und die Decksicht aus Eisentitanat gebildet wird.

Bei einer dritten Ausführungsform besitzt das Pigment einen dreischichtigen Aufbau, wobei auf einem Kern aus plättchenförmigem Titandixoid eine Schicht aus Eisentitanat und eine Deckschicht aus Eisenoxid folgt. In einer besonderen Ausführungsform besitzt dieses Pigment eine zusätzliche Eisentitanatschicht und eine Titandioxidschicht als Deckschicht.

Weiterhin wird diese Aufgabe gemäß der Erfindung gelöst durch ein Verfahren zur Herstellung eines erfindungsgemäßen Pigmentes, wobei das Pigment nur aus Eisentitanat besteht, indem
- eine wäßrige Lösung einer thermisch hydrolysierbaren Titanverbindung als dünner Film auf ein endloses Band aufgebracht wird, wobei die Filmdicke so eingestellt wird, daß sich das für die Pseudobrookit-oder Ilmenitbildung notwendige stöchiometrische Verhältnis zwischen Eisen und Titan einstellt,
- der flüssige Film durch Trocknung verfestigt wird und dabei das Titandioxid durch eine chemische Reaktion aus der Lösung entwickelt wird,
- die entstandene Schicht anschließend vom Band abgelöst und gewaschen wird,
- die erhaltenen Titandioxidplättchen nach oder ohne Zwischentrocknung in Wasser suspendiert und mit Eisenoxid beschichtet werden, wobei die Schichtdicke so eingestellt wird, daß das für die Pseudobrookit- oder Ilmenitbildung notwendige stöchiometrische Verhältnis zwischen Eisen und Titan erhalten wird,
- die beschichteten Partikel aus der wäßrigen Suspension abgetrennt und getrocknet werden und
- in einer oxidierenden oder reduzierenden Atmosphäre bei mindestens 500 °C kalziniert werden.

Weiterhin wird diese Aufgabe gemäß der Erfindung gelöst durch ein Verfahren zur Herstellung eines erfindungsgemäßen Pigments, wobei das Pigment aus einem Kern aus plättchenförmigen Titandioxid und einer Deckschicht aus Eisentitanat gebildet wird, indem
- eine wäßrige Lösung einer thermisch hydrolysierbaren Titanverbindung als dünner Film auf ein endloses Band aufgebracht wird, wobei die Filmdicke so eingestellt wird, daß sowohl für die llmenit- bzw. Pseudobrookitbildung ein Überschuß an Titandioxid vorliegt,
- der flüssige Film durch Trocknung verfestigt wird und dabei das Titandioxid durch eine chemische Reaktion aus der Lösung entwickelt wird,
- die entstandene Schicht anschließend vom Band abgelöst und gewaschen wird,
- die erhaltenen Titandioxidpartikel nach oder ohne Zwischentrocknung in Wasser suspendiert und mit Eisenoxid beschichtet werden, wobei die Schichtdicke so eingestellt wird, daß sowohl für Imenit- bzw. Pseudobrookitbildung ein Überschuß an Titandioxid vorliegt,
- die beschichteten Partikel aus der wäßrigen Suspension abgetrennt und getrocknet werden und
- in einer oxidierenden oder reduzierenden Atmosphäre bei mindestens 500 °C kalziniert werden.

Weiterhin wird diese Aufgabe gemäß der Erfindung gelöst durch ein Verfahren zur Herstellung eines erfindungsgemäßen Pigments, wobei das Pigment dreischichtig ist und auf einem Kern aus plättchenförmigen Titan dioxid eine Schicht aus Eisentitanat und eine Deckschicht aus Eisenoxid folgt, indem
- eine wäßrige Lösung einer thermisch hydrolisierbaren Titanverbindung als dünner Film auf ein endloses Band aufgebracht wird, wobei die Filmdicke so eingestellt wird, daß sich eine Schichtdicke der TiO₂-Schicht von mindestens 40 nm ergibt,
- der flüssige Film durch Trocknung verfestigt wird und dabei das Titandioxid durch eine chemische Reaktion aus dem Precursor entwickelt wird,
- die entstandene Schicht anschließend vom Band abgelöst und gewaschen wird,
- die erhaltenen Titandioxidpartikel nach oder ohne Zwischentrocknung in Wasser suspendiert und mit Eisenoxid beschichtet werden, wobei die Eisenoxidfällung so vorgenommen wird, daß sich nach dem Trocknen eine Schichtdicke von mindestens 20 nm ergibt
- die beschichteten Partikel aus der wäßrigen Suspension abgetrennt und getrocknet werden und
- in einer oxidierenden oder reduzierenden Atmosphäre bei mindestens 500 °C kalziniert werden.

In einer besonderen Ausführungsform besitzt das Pigment eine zusätzliche Eisentitanatschicht und eine Deckschicht aus Titandioxid, indem auf die Eisenoxidschicht noch eine Titandioxidschicht aufgefällt wird, wobei die Titandioxidfällung so vorgenommen wird, daß sich nach dem Trocknen eine Schichtdicke von mindestens 40 nm ergibt.

Gegenstand der Erfindung ist außerdem die Verwendung der erfindungsgemäßen Pigmente zur Pigmentierung von Lacken, Druckfarben, Kunststoffen, Kosmetika und Glasuren für Keramiken und Gläser. Hierfür können sie auch als Mischungen mit handelsüblichen Pigmenten, beispielsweise anorganischen und organischen Absorptionspigmenten, Metalleffektpigmenten und LCP-Pigmenten, eingesetzt werden.

Die Herstellung der erfindungsgemäßen Pigmente erfolgt in einem mehrstufigen Verfahren. In der ersten Stufe wird zunächst das Substrat in Form von Titandioxidplättchen mit Hilfe eines endlosen Bandes hergestellt. In einer zweiten Stufe wird auf das Substrat Eisenoxid aufgefällt, wobei gegebenenfalls noch eine Schicht aus Titandioxid auf die Eisenoxidschicht aufgefällt wird. In einer abschließenden Stufe wird das Pigment unter oxidierenden oder reduzierenden Bedingungen kalziniert, wodurch sich die endgültige Schichtstruktur des Pigmentes ausbildet.

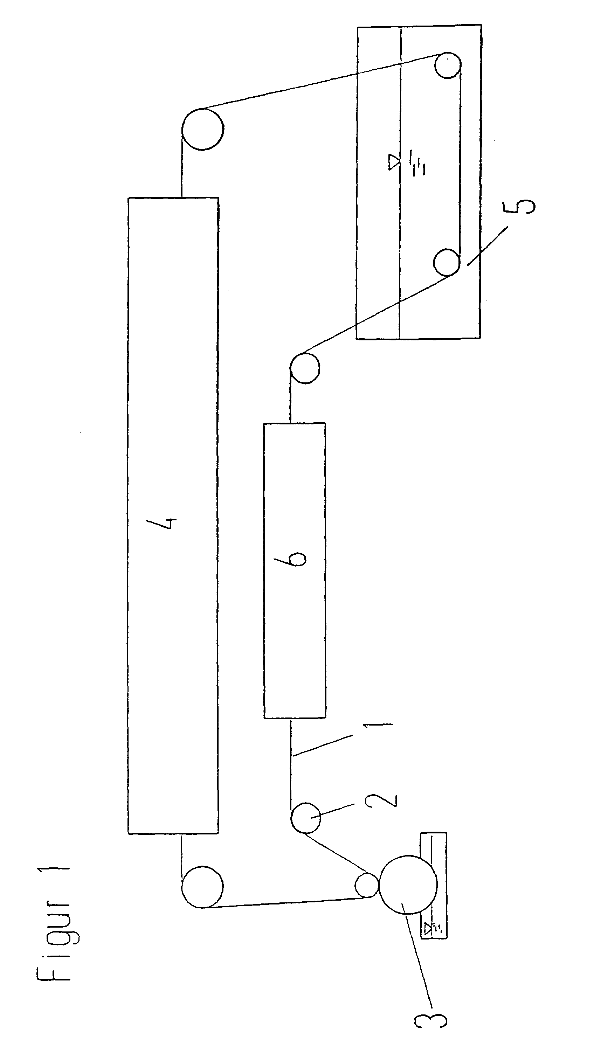

Zunächst soll an Hand von Figur 1 das Bandverfahren erläutert werden.

Das endlose Band 1, welches über ein Rollensystem 2 geführt wird, durchläuft ein Auftragswerk 3, wo es mit einem dünnen Film des Precursors beschichtet wird. Als geeignete Auftragswerke können Walzenauftragswerke sowie Fließer eingesetzt werden. Die Bandgeschwindigkeit liegt zwischen 2 und 400 m/min, vorzugsweise 5 - 200 m/min.

Um eine gleichmäßige Benetzung des Kunststoffbandes zu erzielen, ist es zweckmäßig, der Beschichtungslösung ein handelsübliches Netzmittel zuzusetzen oder die Bandoberfläche durch Beflammung, Koronabehandlung oder lonisation zu aktivieren.

Anschließend durchläuft das beschichtete Band eine Trockenstrecke 4 in der die Schicht bei Temperaturen zwischen 30 und 200 °C getrocknet wird. Als Trockner können zum Beispiel handelsübliche Infrarot-, Umluftdüsen- und UV-Trockner eingesetzt werden.

Nach dem Passieren der Trockenstrecke wird das Band durch die Ablösebäder 5 mit einem geeignetem Ablösemedium, zum Beispiel vollentsalztem Wasser geführt, wo die getrocknete Schicht vom Band entfernt wird. Der Ablösevorgang wird hierbei durch zusätzliche Vorrichtungen, zum Beispiel Düsen, Bürsten oder Ultraschall, unterstützt.

In einem Nachtrockner 6 wird das Band vor der erneuten Beschichtung getrocknet.

Das endlose Band sollte aus einem chemisch und thermisch beständigem Kunststoff sein, um eine ausreichende Standzeit und hohe Trocknungstemperaturen zu gewährleisten. Hierfür sind Materialien wie Polyethylenterephthalat (PET) oder andere Polyester und Polyacrylate geeignet.

Die Folienbreite liegt typischerweise zwischen einigen Zentimetern bis zu mehreren Metern. Die Dicke beträgt zwischen 10 µm bis zu einigen mm, wobei diese beiden Parameter im Hinblick auf die jeweiligen Anforderungen optimiert werden.

Weitere Einzelheiten zu kontinuierlichen Bandverfahren sind aus US 3 138 475, EP 0 240 952 und WO 93/08 237 bekannt.

Die vom Band abgelösten Titandioxidplättchen werden in einer zweiten Verfahrensstufe ohne vorherige Zwischentrocknung mit Eisenoxid nach bekannten Verfahren beschichtet. Dabei kann sowohl von Eisen(III)-salzen ausgegangen werden, wie es zum Beispiel in US 3 087 828 und US 3 087 829 beschrieben ist, als auch von Eisen(III)-salzen, wie in US 3 874 890 beschrieben, wobei der zunächst gebildete Überzug von Eisen(II)-hydroxid zum Eisen(III)-oxidhydrat oxidiert wird. Bevorzugt wird von Eisen(III)-salzen ausgegangen. Hierzu wird einer wäßrigen Suspension der Titandioxidplättchen bei einer Temperatur von 60 bis 90 °C und bei einem pH-Wert von 2,5 bis 4,5 eine Eisen(lll)-chloridlösung zudosiert. Der pH-Wert wird durch gleichzeitige Dosierung von 32%iger Natronlauge konstant gehalten.

Gegebenenfalls wird auf die Eisenoxidhydratschicht anschließend noch eine Titandioxidhydratschicht nach bekannten Verfahren aufgefällt. Bevorzugt wird das in US 3 553 001 beschriebene Verfahren verwendet.

Dabei wird zu auf etwa 50-100 °C insbesondere 70-80 °C erhitzten Suspension der mit Eisenoxidhydrat beschichteten Titandioxidplättchen langsam eine wäßrige Titansalzslösung zugegeben und es wird durch gleichzeitiges Zudosieren einer Base, wie z.B. wäßrige Ammoniaklösung oder eine wäßrige Alkalilauge, ein weitgehend konstanter pH-Wert von etwa 0,5-5, insbesondere etwa 1,5-2,5 eingehalten. Sobald die gewünschte Schichtdicke der TiO₂-Fällung erreicht ist, wird die Zugabe der Titansalzlösung gestoppt.

Dieses, auch als Titrationsverfahren bezeichnete Verfahren zeichnet sich dadurch aus, daß ein Überschuß an Titansalz vermieden wird. Das wird dadurch erreicht, daß man pro Zeiteinheit nur eine solche Menge der Hydrolyse zuführt, wie sie für eine gleichmäßige Beschichtung mit dem hydratisierten TiO₂ erforderlich ist und wie pro Zeiteinheit von der verfügbaren Oberfläche der zu beschichtenden Teilchen aufgenommen werden kann. Es entstehen deshalb keine hydratisierten Titandioxidteilchen, die nicht auf der zu beschichtenden Oberfläche niedergeschlagen sind. Die pro Minute zugesetzte Titansalzmenge liegt dabei in der Größenordnung von etwa 0,01 bis 2·10⁻⁴ Mol Titansalz pro Quadratmeter zu belegender Oberfläche.

In einer dritten Verfahrensstufe werden die mit Eisenoxidhydrat und gegebenenfalls zusätzlich mit Titandioxidhydrat beschichteten Titandioxidplättchen nach Abtrennung aus der Suspension, Waschen und Trocknen bei 500 bis 950 °C, vorzugsweise bei 800 bis 900 °C unter oxidierenden oder reduzierenden Bedingungen kalziniert. Unter diesen Bedingungen diffundiert Eisen in die TiO₂-Schicht, wobei die Anwesenheit von Sauerstoff Pseudobrookit und bei Anwesenheit eines Reduktionsmittels, beispielsweise Wasserstoff, Ilmenit gebildet wird. Bei entsprechender Wahl der Schichtdicke von Eisenoxid und Titandioxid wird ein Pigment erhalten, das ausschließlich aus Eisentitanat besteht.

Das ist dann der Fall, wenn das für die Pseudobrookit- oder Ilmenitbildung notwendige stöchiometrische Verhältnis zwischen Eisen und Titan eingestellt wird.

Liegt dagegen der Anteil des Eisenoxids unter dem stöchiometrischen Verhältnis, erhält man ein Pigment mit einem Kern aus plättchenförmigem Titandioxid und einer Deckschicht aus Eisentitanat.

Wird ein 3-Schichtaufbau TiO₂/Eisentitanat/Fe₂O₃ oder ein 5-Schichtaufbau TiO₂/Eisentitanat/Fe₂O₃/Eisentitanat/TiO₂ des erfindungsgemäßen Pigmentes gewünscht, müssen sowohl das Titandioxidplättchen als auch die aufgefällte Eisenoxidhydratschicht und die gegebenenfalls noch aufgefällte Titandioxidhydratschicht bestimmte Mindeststärken besitzen.

Schichtdicken der Titandioxidhydratschicht beziehungsweise des Titandioxidplättchens von 40 bis 200 nm und insbesondere von 40 bis 150 nm sind bevorzugt.

Wesentlich für den 3- oder 5-Schichtenaufbau ist jedoch insbesondere auch die Schichtdicke der aufgefällten Fe₂O₃-Schicht. Diese sollte in jedem Fall so groß sein, daß nach dem Glühen und der dabei stattfindenden Bildung einer Zwischenschicht von Pseudobrookit oder Ilmenit noch eine reine Fe₂O₃-Schicht von mindestens etwa 15 nm und bevorzugt Schichten mit etwa 15 bis etwa 50 nm, insbesondere solche von etwa 20 bis etwa 40 nm.

Weitere Angaben zur Herstellung eines Mehrschichtenpigments sind US 4 744 832 entnehmbar.

Es ist weiterhin möglich, die Pigmente einer Nachbeschichtung oder Nachbehandlung zu unterziehen, die die Licht-, Wetter- und chemische Stabilität weiter erhöht oder die Handhabung des Pigments, insbesondere die Einarbeitung in unterschiedliche Medien, erleichtert. Als Nachbeschich tung bzw. Nachbehandlung kommen beispielsweise die in den DE-PS 22 15 191, DE-OS 31 51 354, DE-OS 32 35 017 oder DE-OS 33 34 598 beschriebenen Verfahren in Frage. Aufgrund der bereits ohne diese zusätzlichen Maßnahmen sehr guten Eigenschaften der erfindungsgemäßen Pigmente machen diese gegebenenfalls noch aufgebrachten Stoffe nur etwa 0-5, insbesondere 0-3 Gew.% des gesamten Pigments aus.

Das erfindungsgemäße Pigment kann noch zusätzlich mit schwerlöslichen, fest haftenden anorganischen oder organischen Farbmitteln beschichtet werden. Bevorzugt werden Farblacke und insbesondere Aluminiumfarblacke verwendet. Dazu wird eine Aluminiumhydroxidschicht aufgefällt, die in einem zweiten Schritt mit einem Farblack verlackt wird. Das Verfahren ist in DE 24 29 762 und DE 29 28 287 näher beschrieben.

Bevorzugt ist auch eine zusätzliche Beschichtung mit Komplexsalzpigmenten, insbesondere Cyanoferratkomplexen, wie zum Beispiel Berliner Blau und Tumbulls Blau, wie sie in EP 0 141 173 und DE 23 13 332 beschrieben ist.

Das erfindungsgemäße Pigment kann auch mit organischen Farbstoffen und insbesondere mit Phthalocyanin- oder Metallphthalocyanin- und/oder Indanthrenfarbstoffen nach DE 40 09 567 beschichtet werden. Dazu wird eine Suspension des Pigmentes in einer Lösung des Farbstoffes hergestellt und diese dann mit einem Lösungsmittel zusammengebracht, in welchem der Farbstoff schwer löslich oder unlöslich ist.

Weiterhin können auch Metallchalkogenide bzw. Metalchalkogenidhydrate und Ruß für eine zusätzliche Beschichtung eingesetzt werden.

Das erfindungsgemäße Pigment stellt bezüglich der Dicke den Idealzustand dar, der bei Perlglanzpigmenten maximal erreichbar ist, da es nur aus optisch funktionellen Schichten besteht und ein sonst übliches Trägermaterial, wie beispielsweise Glimmer oder Glasplättchen, das nicht zum optischen Effekt beiträgt, fehlt. Bedingt durch die Dicke des Glimmers besitzen Glimmerpigmente eine bis um den Faktor 25 größere Dicke bei gleicher Dicke der funktionellen Schichten. Bezüglich der technischen Anwendungen ergeben sich hieraus intrinsische Vorteile, die von keinem anderen konventionellen Perlglanzpigment erreicht werden können. Beispielsweise können Lackschichten dünner ausgeführt werden und die notwendige Pigmentmenge kann reduziert werden, weil aufgrund des Fehlens des "Füllstoffs" Trägermaterial die Pigmente optisch aktiver sind.

Die im folgenden angegebenen Beispielen sollen die Erfindung erläutern, ohne sie zu begrenzen.

### Beispiel 1

15 g TiO₂-Plättchen (Schichtdicke ca. 60 nm) werden in 2000 ml vollentsalztem Wasser (VE-Wasser) suspendiert und auf 75 °C erhitzt.

Der pH-Wert der Suspension wird mit 10%iger Salzsäure auf 3,0 eingestellt. Nun werden 610 g einer wäßrigen 10%igen FeCl₃-Lösung bei 75 °C innerhalb von 4 h unter Konstanthaltung des pH-Wertes durch gleichzeitige Zugabe von 32%iger Natronlauge zudosiert. Zur Vervollständigung der Fällung wird noch 45 min bei 75 °C nachgerührt.

Anschließend läßt man auf Raumtemperatur abkühlen, filtriert das erhaltene rot-braune Pigment ab, wäscht mit VE-Wasser salzfrei und trocknet bei 100 °C. Abschließend wird 45 min bei 850 °C geglüht. Man erhält ein kupferrotes Perglanzpigment aus Pseudobrookit mit goldener Interferenzfarbe.

### Beispiel 2

38 g TiO₂-Plättchen werden in 2000 ml VE-Wasser suspendiert und auf 75 °C erhitzt.

Der pH-Wert der Suspension wird mit 10%iger Salzsäure auf 3,0 eingestellt. Nun werden 770 g einer wäßrigen 10%igen FeCl₃-Lösung bei 75 °C innerhalb von 5 h unter Konstanthaltung des pH-Wertes durch gleichzeitige Zugabe von 32%iger Natronlauge zudosiert. Zur Vervollständigung der Fällung wird noch 45 min bei 75 °C nachgerührt.

Anschließend läßt man auf Raumtemperatur abkühlen, filtriert das erhaltene Pigment ab, wäscht mit VE-Wasser salzfrei und trocknet bei 100 °C. Abschließend wird 45 min bei 850 °C geglüht. Man erhält ein goldfarbenes Perglanzpigment, das aus 25 % Titandioxid und 75 % Pseudobrookit besteht.

### Beispiel 3

20 g des in Beispiel 2 hergestellten, getrockneten Pigments werden in einem Röhrenofen unter Formiergas (N₂/H₂= 95/5) 3 Stunden bei 750 °C geglüht. Man erhält ein blauschwarzes, glänzendes Pigment mit Ilmenitstruktur.

### Beispiel 4

Das in Beispiel 2 hergestellte, getrocknete Pigment wird an Luft min. bei 700 °C geglüht. Man erhält ein rotbraunes Periglanzpigment mit kupferner Interferenzfarbe, das aus einem Titandioxidkern, einer Pseudobrookitschicht und einer Hämatitaußenschicht besteht.

### Beispiel 5

30 g TiO₂-Plättchen werden in 2000 ml VE-Wasser suspendiert und auf 75 °C erhitzt.

Der pH-Wert der Suspension wird mit 10%iger Salzsäure auf 3,0 eingestellt. Nun werden 815 g einer wäßrigen 10%igen FeCl₃-Lösung bei 75 °C innerhalb von 5 h unter Konstanthaltung des pH-Wertes durch gleichzeitige Zugabe von 32%iger Natronlauge zudosiert. Zur Vervollständigung der Fällung wird noch 30 min bei 75 °C nachgerührt.

Nun werden 120 ml einer wäßrigen TiCl₄-Lösung (400 g TiCl₄/l) innerhalb von 60 min hinzudosiert. Der pH-Wert wird während der gesamten Zugabe mit 32%iger NaOH-Lösung bei 2,2 konstant gehalten. Nach beendeter Zugabe wird zur Vervollständigung der Fällung 30 min bei 75 °C nachgerührt.

Anschließend läßt man auf Raumtemperatur abkühlen, filtriert das erhaltene Pigment ab, wäscht mit VE-Wasser salzfrei und trocknet bei 110 °C. Abschließend wird 30 min bei 850 °C geglüht. Man erhält ein goldfarbenes Perglanzpigment mit rötlicher Interferenzfarbe, das aus einem Titandioxidkern, einer Pseudobrookitschicht und einer Titandioxidaußenschicht besteht.

## Patentansprüche

1. Einschichtige oder mehrschichtige titanathaltige Perlglanzpigmente, bestehend aus Eisentitanat und gegebenenfalls Titanoxid und/oder Eisenoxid, erhältlich durch Verfestigung einer wäßrigen Lösung einer thermisch hydrolysierbaren Titanverbindung auf einem endlosen Band, Ablösung der entstandenen Schicht, Beschichtung der erhaltenen Titandioxidplättchen nach oder ohne Zwischentrocknung im Naßverfahren mit Eisenoxid, Trocknung und Kalzination des erhaltenen Materials in einer oxidierenden oder reduzierenden Gasatmosphäre bei mindestens 700 °C, wobei die Schichtdicken der Titandioxid- und Eisenoxidschicht so eingestellt werden, daß entweder das für die Pseudobrookit- oder Ilmenitbildung notwendige stöchiometrische Verhältnis zwischen Eisenoxid und Titandioxid erhalten wird oder ein Überschuß an Eisenoxid oder Titandioxid vorliegt.

2. Perlglanzpigmente nach Anspruch 1, dadurch gekennzeichnet, daß die wäßrige Lösung einer thermisch hydrolysierbaren Titanverbindung eine wäßrige Titantetrachloridlösung ist.

3. Verfahren zur Herstellung der Perlglanzpigmente nach Anspruch 1, wobei die Perlglanzpigmente aus Eisentitanat bestehen, dadurch gekennzeichnet, daß
- eine wäßrige Lösung einer thermisch hydrolysierbaren Titanverbindung als dünner Film auf ein endloses Band aufgebracht wird, wobei die Filmdicke so eingestellt wird, daß sich das für die Pseudobrookit- oder Ilmenitbildung notwendige stöchiometrische Verhältnis zwischen Eisen und Titan einstellt,
- der flüssige Film durch Trocknung verfestigt wird und dabei das Titandioxid durch eine chemische Reaktion aus der Lösung entwickelt wird,
- die entstandene Schicht anschließend vom Band abgelöst und gewaschen wird,
- die erhaltenen Titandioxidplättchen nach oder ohne Zwischentrocknung in Wasser suspendiert und mit Eisenoxid beschichtet werden, wobei die Schichtdicke so eingestellt wird, daß das für die Pseudobrookit- oder Ilmenitbildung notwendige stöchiometrische Verhältnis zwischen Eisen und Titan erhalten wird,
- die beschichteten Partikel aus der wäßrigen Suspension abgetrennt und getrocknet werden und
- in einer oxidierenden oder reduzierenden Atmosphäre bei mindestens 500 °C kalziniert werden.

4. Verfahren zur Herstellung der Periglanzpigmente nach Anspruch 1, wobei die Pigmente aus einem Kern aus plättchenförmigem Titandioxid und einer Deckschicht aus Eisentitanat gebildet werden, dadurch gekennzeichnet, daß
- eine wäßrige Lösung einer thermisch hydrolysierbaren Titanverbindung als dünner Film auf ein endloses Band aufgebracht wird, wobei die Filmdicke so eingestellt wird, daß sowohl für die Ilmenit- bzw. Pseudobrookitbildung ein Überschuß an Titandioxid vorliegt,
- der flüssige Film durch Trocknung verfestigt wird und dabei das Titandioxid durch eine chemische Reaktion aus der Lösung entwickelt wird,
- die entstandene Schicht anschließend vom Band abgelöst und gewaschen wird,
- die erhaltenen Titandioxidpartikel nach oder ohne Zwischentrocknung in Wasser suspendiert und mit Eisenoxid beschichtet werden, wobei die Schichtdicke so eingestellt wird, daß sowohl für Imenit- bzw. Pseudobrookitbildung ein Überschuß an Titandioxid vorliegt,
- die beschichteten Partikel aus der wäßrigen Suspension abgetrennt und getrocknet werden und
- in einer oxidierenden oder reduzierenden Atmosphäre bei mindestens 500 °C kalziniert werden.

5. Verfahren zur Herstellung der Perlglanzpigmente nach Anspruch 1, wobei die Pigmente dreischichtig sind und auf einem Kern aus plättchenförmigem Titandioxid eine Schicht aus Eisentitanat und eine Deckschicht aus Eisenoxid folgt, dadurch gekennzeichnet, daß
- eine wäßrige Lösung einer thermisch hydrolisierbaren Titanverbindung als dünner Film auf ein endloses Band aufgebracht wird, wobei die Filmdicke so eingestellt wird, daß sich eine Schichtdicke der TiO₂-Schicht von mindestens 40 nm ergibt,
- der flüssige Film durch Trocknung verfestigt wird und dabei das Titandioxid durch eine chemische Reaktion aus dem Precursor entwickelt wird,
- die entstandene Schicht anschließend vom Band abgelöst und gewaschen wird,
- die erhaltenen Titandioxidpartikel nach oder ohne Zwischentrocknung in Wasser suspendiert und mit Eisenoxid beschichtet werden, wobei die Eisenoxidfällung so vorgenommen wird, daß sich nach dem Trocknen eine Schichtdicke von mindestens 20 nm ergibt,
- die beschichteten Partikel aus der wäßrigen Suspension abgetrennt und getrocknet werden und
- in einer oxidierenden oder reduzierenden Atmosphäre bei mindestens 500 °C kalziniert werden.

6. Verfahren nach Anspruch 5, dadurch gekennzeichnet, daß auf die Eisenoxidschicht noch eine Titandioxidschicht aufgefällt wird, wobei die Titandioxidfällung so vorgenommen wird, daß sich nach dem Trocknen eine Schichtdicke von mindestens 40 nm ergibt.

7. Verfahren nach mindestens einem der Ansprüche 3 bis 6, dadurch gekennzeichnet, daß das Eisenoxid und das Titandioxid nach Zwischentrocknung der Titandioxidplättchen in einem Wirbelbettreaktor durch CVD aufgebracht werden.

8. Verfahren nach mindestens einem der Ansprüche 3 bis 5, dadurch gekennzeichnet, daß die wäßrige Lösung einer thermisch hydrolysierbaren Titanverbindung eine wäßrige Titantetrachloridlösung ist.

9. Verwendung der Pigmente nach den Ansprüchen 1 und 2 zur Pigmentierung von Lacken, Druckfarben, Kunststoffen, Kosmetika und Glasuren für Keramiken und Gläser.

10. Verwendung nach Anspruch 9, dadurch gekennzeichnet, daß die Pigmente als Mischungen mit handelsüblichen Pigmenten eingesetzt werden.

11. Lacke, Druckfarben, Kunststoffe, Kosmetika, Keramiken und Gläser, welche mit einem Pigment nach den Ansprüchen 1 und 2 pigmentiert sind.

## Claims

1. Single-layer or multilayer titanate-containing pearlescent pigments comprising iron titanate and, if appropriate, titanium oxide and/or iron oxide, which is obtainable by solidification of an aqueous solution of a thermally hydrolysable titanium compound on a continuous belt, detachment of the layer formed, coating of the resulting titanium dioxide platelets, after or without intermediate drying, with iron oxide in a wet process, and drying and calcining of the resulting material in an oxidizing or reducing gas atmosphere at not less than 700°C, the layer thicknesses of the titanium dioxide layer and iron dioxide layer being adjusted such that either the stoichiometric ratio between iron oxide and titanium dioxide necessary for the formation of pseudo-brookite or ilmenite is obtained or an excess of iron oxide or titanium dioxide is present.

2. Pearlescent pigments according to Claim 1, characterized in that the aqueous solution of a thermally hydrolysable titanium compound is an aqueous titanium tetrachloride solution.

3. Process for the preparation of the pearlescent pigments according to Claim 1, wherein the pearlescent pigments comprise iron titanate, characterized in that
- an aqueous solution of a thermally hydrolysable titanium compound is applied as a thin film to a continuous belt, the film thickness being adjusted such that the stoichiometric ratio between iron and titanium necessary for the formation of pseudo-brookite or ilmenite is established,
- the liquid film is solidified by drying, during which the titanium dioxide is developed from the solution by a chemical reaction,
- the layer formed is then detached from the belt and washed,
- the resulting titanium dioxide platelets are suspended in water, after or without intermediate drying, and coated with iron oxide, the layer thickness being adjusted such that the stoichiometric ratio between iron and titanium necessary for the formation of pseudo-brookite or ilmenite is obtained, and
- the coated particles are separated off from the aqueous suspension and dried and
- calcined in an oxidizing or reducing atmosphere at not less than 500°C.

4. Process for the preparation of the pearlescent pigments according to Claim 1, wherein the pigments are formed from a core of plateletlike titanium dioxide and a top layer of iron titanate, characterized in that
- an aqueous solution of a thermally hydrolyzable titanium compound is applied as a thin film to a continuous belt, the film thickness being adjusted such that an excess of titanium dioxide is present for the formation of both ilmenite and pseudo-brookite,
- the liquid film is solidified by drying, during which the titanium dioxide is developed from the solution by a chemical reaction,
- the layer formed is then detached from the belt and washed,
- the resulting titanium dioxide particles are suspended in water, after or without intermediate drying, and coated with iron oxide, the layer thickness being adjusted such that an excess of titanium dioxide is present for the formation of both ilmenite and pseudo-brookite, and
- the coated particles are separated off from the aqueous suspension and dried and
- calcined in an oxidizing or reducing atmosphere at not less than 500°C.

5. Process for the preparation of the pearlescent pigments according to Claim 1, wherein the pigments have three layers and a core of plateletlike titanium dioxide is followed by a layer of iron titanate and a top layer of iron oxide, characterized in that
- an aqueous solution of a thermally hydrolysable titanium compound is applied as a thin film to a continuous belt, the film thickness being adjusted such that a layer thickness of the TiO₂ layer of at least 40 nm results,
- the liquid film is solidified by drying, during which the titanium dioxide is developed from the precursor by a chemical reaction,
- the layer formed is then detached from the belt and washed,
- the resulting titanium dioxide particles are suspended in water, after or without intermediate drying, and coated with iron oxide, the iron oxide precipitation being carried out such that a layer thickness of at least 20 nm results after the drying, and
- the coated particles are separated off from the aqueous suspension and dried and
- calcined in an oxidizing or reducing atmosphere at not less than 500°C.

6. Process according to Claim 5, characterized in that a titanium dioxide layer is also precipitated onto the iron oxide layer, the titanium dioxide precipitation being carried out such that a layer thickness of at least 40 nm results after drying.

7. Process according to at least one of Claims 3 to 6, characterized in that the iron oxide and the titanium dioxide are applied by CVD in a fluidized bed reactor after intermediate drying of the titanium dioxide platelets.

8. Process according to at least one of Claims 3 to 5, characterized in that the aqueous solution of a thermally hydrolysable titanium compound is an aqueous titanium tetrachloride solution.

9. Use of the pigments according to Claims 1 and 2 for pigmenting coatings, printing inks, plastics, cosmetics and glazings for ceramics and glass.

10. Use according to Claim 9, characterized in that the pigments are employed as mixtures with commercially available pigments.

11. Coatings, printing inks, plastics, cosmetics, ceramics and glass pigmented with a pigment according to Claims 1 and 2.

## Revendications

1. Pigments nacrés monocouche ou multicouche contenant des titanates, constitués par du titanate de fer et éventuellement par de l'oxyde de titane et/ ou par de l'oxyde de fer, obtenus par solidification d'une solution aqueuse d'un composé du titane thermiquement hydrolysable sur une bande sans fin, par séparation de la couche obtenue, par revêtement des lamelles de dioxyde de titane obtenues, après ou sans séchage intermédiaire, dans un procédé par voie humide avec de l'oxyde de fer, par séchage et par calcination du matériau obtenu dans une atmosphère de gaz oxydante ou réductrice à au moins 700°C, l'épaisseur des couches de dioxyde de titane et d'oxyde de fer étant ajustée de telle façon que soit l'on obtienne le rapport stoechiométrique entre l'oxyde de fer et le dioxyde de titane, nécessaire à la formation de la pseudobrookite ou de l'ilménite, soit l'on se trouve en présence d'un excès d'oxyde de fer ou de dioxyde de titane.

2. Pigments nacrés selon la revendication 1, caractérisés en ce que la solution aqueuse d'un composé du titane thermiquement hydrolysable est une solution aqueuse de tétrachlorure de titane.

3. Procédé pour la préparation des pigments nacrés selon la revendication 1, les pigments nacrés étant constitués par du titanate de fer, caractérisé en ce que :
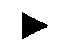 l'on applique une solution aqueuse d'un composé du titane thermiquement hydrolysable sous la forme d'un film mince sur une bande sans fin, l'épaisseur du film étant ajustée de telle façon que s'établisse le rapport stoechiométrique entre le fer et le titane, nécessaire à la formation de la pseudobrookite ou de l'ilménite,
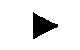 l'on solidifie le film liquide par séchage, le dioxyde de titane se formant à partir de la solution par une réaction chimique
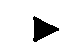 l'on sépare ensuite de la bande la couche obtenue et on la lave,
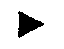 l'on met en suspension dans l'eau les lamelles de dioxyde de titane obtenues, après ou sans séchage intermédiaire, et on les revêt avec de l'oxyde de fer, l'épaisseur de la couche étant ajustée de telle façon que l'on obtienne le rapport stoechiométrique entre le fer et le titane, nécessaire à la formation de la pseudobrookite ou de l'ilménite,
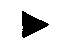 l'on sépare de la suspension aqueuse les particules revêtues et on les sèche et
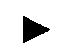 on les calcine à au moins 500°C dans une atmosphère oxydante ou réductrice.

4. Procédé pour la préparation des pigments nacrés selon la revendication 1, les pigments étant formés d'un noyau en dioxyde de titane lamellaire et d'une couche de recouvrement en titanate de fer, caractérisé en ce que :
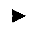 l'on applique une solution aqueuse d'un composé du titane thermiquement hydrolysable sous la forme d'un film mince sur une bande sans fin, l'épaisseur du film étant ajustée de telle façon que l'on soit en présence d'un excès de dioxyde de titane, aussi bien pour la formation de l'ilménite que pour celle de la pseudobrookite,
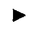 l'on solidifie le film liquide par séchage, le dioxyde de titane se formant à partir de la solution par une réaction chimique,
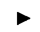 l'on sépare ensuite de la bande la couche obtenue et on la lave,
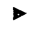 l'on met en suspension dans l'eau les particules de dioxyde de titane obtenues, après ou sans séchage intermédiaire, et on les revêt avec de l'oxyde de fer, l'épaisseur de la couche étant ajustée de telle façon que l'on soit en présence d'un excès de dioxyde de titane, aussi bien pour la formation de l'ilménite que pour celle de la pseudobrookite,
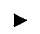 l'on sépare de la suspension aqueuse les particules revêtues et on les sèche et
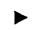 les calcine à au moins 500°C dans une atmosphère oxydante ou réductrice.

5. Procédé pour la préparation des pigments nacrés selon la revendication 1, les pigments comportant trois couches, le noyau en dioxyde de titane lamellaire étant suivi d'une couche de titanate de fer et d'une couche de recouvrement en oxyde de fer, caractérisé en ce que :
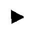 l'on applique une solution aqueuse d'un composé du titane thermiquement hydrolysable sous la forme d'un film mince sur une bande sans fin, l'épaisseur du film étant ajustée de telle façon que l'on obtienne une couche de TiO₂ d'une épaisseur d'au moins 40 nm,
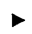 l'on solidifie le film liquide par séchage, le dioxyde de titane se formant à partir du précurseur par une réaction chimique,
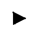 l'on sépare ensuite de la bande la couche obtenue et on la lave,
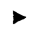 l'on met en suspension dans l'eau les particules de dioxyde de titane, après ou sans séchage intermédiaire, et on les revêt avec de l'oxyde de fer, la précipitation de l'oxyde de fer étant effectuée de telle façon que l'on obtienne après séchage une épaisseur de couche d'au moins 20 nm,
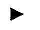 l'on sépare de la suspension aqueuse les particules revêtues et on les sèche et
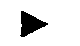 on les calcine à au moins 500°C dans une atmosphère oxydante ou réductrice.

6. Procédé selon la revendication 5, caractérisé en ce que l'on précipite sur la couche d'oxyde de fer encore une couche de dioxyde de titane, la précipitation de l'oxyde de titane étant effectuée de telle façon que l'on obtienne après le séchage une épaisseur de couche d'au moins 40 nm.

7. Procédé selon au moins l'une des revendications 3 à 6, caractérisé en ce que l'on applique l'oxyde de fer et le dioxyde de titane, après séchage intermédiaire des lamelles de dioxyde de titane, par CVD dans un réacteur à lit fluidisé.

8. Procédé selon au moins l'une des revendications 3 à 5, caractérisé en ce que la solution aqueuse d'un composé du titane thermiquement hydrolysable est une solution aqueuse de tétrachlorure de titane.

9. Utilisation des pigments selon les revendications 1 et 2 pour la pigmentation des vernis, encres d'imprimerie, matières plastiques, cosmétiques et glaçures pour céramiques et verres.

10. Utilisation selon la revendication 9, caractérisée en ce que l'on utilise les pigments sous forme de mélanges avec des pigments courants dans le commerce.

11. Vernis, encres d'imprimerie, matières plastiques, cosmétiques, céramiques et verres pigmentés avec un pigment selon les revendications 1 et 2.
